# EUROPEAN PATENT APPLICATION

(11) **EP 1 666 061 A1**
(43) Date of publication of application: **07.06.2006**
(21) Application number: 04773070.0
(22) Date of filing: 08.09.2004
(51) Int. Cl.: A61K 39/395, A61P 25/28

(54) **USE OF ANTIBODY**

(30) Priority: 09.09.2003 JP 2003317443
(71) Applicant: Takeda Pharmaceutical Company Limited, Osaka-shi, Osaka 5408645 (JP)
(72) Inventor: SHOJI, Mikio, Okayama-shi, Okayama 7038275 (JP); ASAMI, Asano, Tsukuba-shi, Ibaraki 3050047 (JP); SUZUKI, Nobuhiro, Mino-shi, Osaka 5620001 (JP)
(74) Representative: von Kreisler Selting Werner
(86) International application number: PCT/JP2004/013397
(87) International publication number: WO 2005/025616

(57) **Abstract**

The present invention intended to provide an agent for preventing/treating Alzheimer's disease. More specifically, the present invention provides an agent for preventing/treating Alzheimer's disease, etc., which comprises a monoclonal antibody specifically reacting with a partial peptide at the C-terminal region of a β-amyloid or its derivatives.

## Description

### TECHNICAL FIELD

The present invention relates to a preventing/treating agent or a diagnostic agent for Alzheimer's disease, etc. More particularly, the present invention relates to a preventing/treating agent or a diagnostic agent for Alzheimer's disease, etc., which comprises a monoclonal antibody specifically reacting with a partial peptide at the C-terminal region of a β-amyloid (Aβ) or its derivatives.

### BACKGROUND ART

Senile dementia caused by Alzheimer's disease has raised a serious social problem, and it has been desired to establish preventive/therapeutic strategies for Alzheimer's disease at an early stage. As lesions characteristic of the brains of patients with Alzheimer's disease, the excessive formation of senile plaque amyloids and neurofibrillary degeneration are known. Among these, one of the major components of senile plaques is a β-amyloid or its derivatives.

The β-amyloid is a peptide composed of about 40 to 43 amino acids and is encoded in the vicinity of the transmembrane domain of an amyloid precursor protein (hereinafter abbreviated as APP). Amino acid sequences of the β-amyloid are shown below.
β-Amyloid (1-38) = SEQ ID NO: 1
β-Amyloid (1-39) = SEQ ID NO: 2
β-Amyloid (1-40) = SEQ ID NO: 3
β-Amyloid (1-41) = SEQ ID NO: 4
β-Amyloid (1-42) = SEQ ID NO: 5
β-Amyloid (1-43) = SEQ ID NO: 6

Recently, it is further reported that of these β-amyloids, β-amyloid (1-42) is mainly deposited in the cerebral parenchyma (senile plaques) at an early stage, whereas in the cerebral blood vessel β-amyloid (1-40) is mainly deposited (amyloid angiopathy) [Arch. Biochem. Biophys., 301, 41-53, 1993]. It is further suggested that peptides containing the C-terminal residues such as β-amyloid (1-42), β-amyloid (26-42), β-amyloid (26-43), β-amyloid (34-42), etc. become the seed to form deposits of water-soluble β-amyloid (1-40), etc. [Biochemistry 32, 4693-4697, 1993]. Thus, the deposits of β-amyloid (x-42) are thought to be the most important pathogenesis of Alzheimer's disease. From these reports, it is considered that the difference in deposit patterns between β-amyloid (x-40) and β-amyloid (x-42) would be largely related to Alzheimer's disease.

For this reason, differential determination of β-amyloid (x-40) and β-amyloid (x-42) with high sensitivity is considered important. WO 94/17197 discloses an antibody specifically reacting with a partial peptide at the C-terminal region of a β-amyloid or its derivatives and a method for assaying the β-amyloid having a C-terminal hydrophobic region, which comprises using the antibody. However, any direct effect of preventing/treating Alzheimer's disease by the antibody is not known.

On the other hand, direct treatment of Alzheimer's disease with a β-amyloid vaccine is reported in, e.g., Nature, 400, 173-177, 1999, Nature Medicine, 9 (4), 448-452, 2003, etc. The direct treatment reportedly brought success in reducing senile plaques, improving learning ability, etc. in preclinical trials using human APP transgenic mice, etc., and such was expected to be a breakthrough treatment. Nonetheless, the β-amyloid vaccine caused unexpectedly serious side effects in the latest phase II clinical trials. The major cause of the serious side effects even resulting in deaths is considered due to the onset of encephalitis caused by infiltration of CD4⁺ lymphocytes into the brain, which is not observed in regular Alzheimer's disease.

As a result of immunotherapy, senile plaques deposited in the brain dramatically decreased and improvement in cognitive ability was observed, which has been confirmed also in human [Neuron, 38, 547-555, 2003]. As an option to avoid the side effects while highlighting this advantage, passive immunization by peripheral administration of an anti β-amyloid antibody is proposed and has been studied on an animal level using a monoclonal antibody [Science, 295, 2264-2267, 2002, Nature Neuroscience, 5, 452-457, 2002]. However, it is reported that in the studies using a model mouse with cerebral amyloid angiopathy (CAA), administration of anti-N-terminal β-amyloid antibody worsened CAA-associated cerebral hemorrhage [Science, 298, 1379, 2002].

An increased blood level of β-amyloid was reported in experiments of passive immunization on an animal level by using an antibody, which has an N-terminal region or a mid-region of β-amyloid as its epitope [Science, 295, 2264-2267, 2002, Nature Neuroscience, 5, 452-457, 2002, Nature Medicine, 8, 1263-1269, 2002 and Proceedings of the National Academy of Sciences of the USA, 100, 2023-2028, 2003]. However, there is no report of immunotherapy whereby the blood level of highly aggregative β-amyloid (x-42) is elevated specifically.

### DISCLOSURE OF INVENTION

It is pointed out that the β-amyloid is a potential pathologic agent in Alzheimer's disease but despite a deep interest in β-amyloid, it is the actual situation that any effective preventive/therapeutic strategy has not been established. Further for immunotherapy of Alzheimer's disease, antibodies that specifically react with the N-terminal region or mid-region of the β-amyloid or with a partial peptide of the C-terminal region of β-amyloid (x-40) is at risk of inducing inflammation by, e.g., β-amyloid (x-40) (CAA) deposited on the cerebrovascular wall, and thus leading to cerebral hemorrhage, etc. In view of these problems, the immunotherapy is not necessarily a fully safe preventive/therapeutic strategy for Alzheimer's disease.

As above, it is the current status that it has been earnestly desired to provide a more effective and safe method for preventing/treating Alzheimer's disease.

The present inventors have made extensive investigations to solve the foregoing problems and as a result, have found that when monoclonal antibody BC-05a specifically reacting with a partial peptide at the C-terminal region of a β-amyloid or its derivatives was subcutaneously administered to model mice with Alzheimer's disease, deposits of β-amyloid in the brain were unexpectedly prevented and the blood level of β-amyloid (x-42) alone was selectively increased without increasing the blood level of β-amyloid (x-40). Based on these findings, the inventors have continued further investigations and come to accomplish the present invention.

That is, the present invention provides the following features:
(1) An agent for preventing/treating Alzheimer's disease, mild cognitive impairment or cerebral amyloid angiopathy, comprising a monoclonal antibody, which specifically reacts with a partial peptide at the C-terminal region of a β-amyloid or a derivative thereof and does not recognize a partial peptide having the amino acid sequence represented by SEQ ID NO: 8;
(2) The agent according to (1), which is an agent for preventing/treating Alzheimer's disease;
(3) The agent according to (1), wherein said antibody is an antibody which does not recognize a partial peptide having the amino acid sequence represented by SEQ ID NO:9;
(4) The agent according to (1), wherein said antibody is an antibody which recognizes a partial peptide having the amino acid sequence represented by SEQ ID NO: 9;
(5) The agent according to (1), wherein said β-amyloid is a peptide having the amino acid sequence represented by SEQ ID NO: 1, SEQ ID NO: 2, SEQ ID NO: 3, SEQ ID NO: 4, SEQ ID NO: 5 or SEQ ID NO: 6;
(6) The agent according to (1), wherein said β-amyloid is a peptide having the amino acid sequence represented by SEQ ID NO: 5;
(7) The agent according to (1), wherein said derivative of the β-amyloid is a peptide having the amino acid sequence from the 2nd to the 42nd in the amino acid sequence represented by SEQ ID NO: 5, a peptide having the amino acid sequence from the 3rd to the 42nd in the amino acid sequence represented by SEQ ID NO: 5, in which the N-terminal glutamic acid is converted into pyroglutamic acid, or a peptide having the amino acid sequence from the 4th to the 42nd in the amino acid sequence represented by SEQ ID NO: 5;
(8) The agent according to (1), wherein said derivative of the β-amyloid is a peptide having an amino acid sequence lacking the 1st to the 10th amino acid sequence in each of the amino acid sequences represented by SEQ ID NO: 1 through SEQ ID NO: 6, in which the N-terminal glutamic acid is converted into pyroglutamic acid;
(9) The agent according to (1), wherein said partial peptide at the C-terminal region of the β-amyloid or a derivative thereof is a partial peptide having an amino acid sequence beginning from the 25th amino acid from the N-terminal amino acid of each β-amyloid;
(10) The agent according to (1), wherein said antibody is an antibody which does not recognize a partial peptide having the amino acid sequence represented by SEQ ID NO:7;
(11) The agent according to (1), wherein said antibody is an antibody which recognizes β-amyloid (1-42) having the amino acid sequence represented by SEQ ID NO: 5;
(12) The agent according to (1), wherein said antibody is monoclonal antibody BA-27a, which is producible from the hybridoma indicated by BA-27 (FERM BP-4139);
(13) The agent according to (1), wherein said antibody is monoclonal antibody BC-05a, which is producible from the hybridoma indicated by BC-05 (FERM BP-4457);
(14) The agent according to (1), wherein said antibody passes through a blood-brain barrier;
(15) The agent according to (14), wherein said antibody is an antibody capable of drawing the β-amyloid out of senile plaques formed;
(16) The agent according to (1), which is an agent for suppressing aggregation or deposition of the β-amyloid in the brain;
(17) The agent according to (1), which is capable of specifically increasing the blood level of a peptide having the amino acid sequence represented by SEQ ID NO: 5;
(18) The agent according to (1), wherein said antibody is an antibody which does not pass through a blood-brain barrier;
(19) The agent according to (18), wherein said antibody is an antibody capable of capturing the peripheral β-amyloid in the periphery;
(20) A method for preventing/treating Alzheimer's disease, mild cognitive impairment or cerebral amyloid angiopathy, which comprises administering to a mammal an effective dose of a monoclonal antibody, which specifically reacts with a partial peptide at the C-terminal region of a β-amyloid or a derivative thereof and does not recognize a partial peptide having the amino acid sequence represented by SEQ ID NO: 8;
(21) Use of a monoclonal antibody, which specifically reacts with a partial peptide at the C-terminal region of a β-amyloid or a derivative thereof and does not recognize a partial peptide having the amino acid sequence represented by SEQ ID NO: 8, to produce an agent for preventing/treating Alzheimer's disease, mild cognitive impairment or cerebral amyloid angiopathy;
(22) The agent according to (1), wherein said antibody recognizes β-amyloid (1-42) having the amino acid sequence represented by SEQ ID NO: 5 but does not recognize any of β-amyloid (1-38) having the amino acid sequence represented by SEQ ID NO: 1, β-amyloid (1-39) having the amino acid sequence represented by SEQ ID NO: 2 and β-amyloid (1-40) having the amino acid sequence represented by SEQ ID NO: 3; and the like.

### BEST MODE FOR CARRYING OUT THE INVENTION

In the anti-β-amyloid antibody-producing hybridoma cells used in the present invention, BA-27 has been deposited in the Institute for Fermentation (IFO), located at 2-17-85, Juso-honmachi, Yodogawa-ku, Osaka-shi, Osaka, (postal code: 532-8686), Japan, under the accession number IFO 50387 since December 22, 1992 and in the National Institute of Bioscience and Human-Technology (NIBH), Ministry of International Trade and Industry [currently International Patent Organism Depository, National Institute of Advanced Industrial Science and Technology], located at Central 6, 1-1-1 Higashi, Tsukuba, Ibaraki, (postal code: 305-8566), Japan, under the accession number FERM BP-4139 since January 7, 1993.

Further in the anti-β-amyloid antibody producing hybridoma cells used in the present invention, BC-05 has been deposited in the National Institute of Bioscience and Human-Technology (NIBH), Ministry of International Trade and Industry [currently, International Patent Organism Depository, National Institute of Advanced Industrial Science and Technology], located at Central 6, 1-1-1 Higashi, Tsukuba, Ibaraki, (postal code: 305-8566), Japan, under the accession number FERM BP-4457 since November 2, 1993.

In this specification, the antibody obtained from each hybridoma cells is represented by adding the suffix "a" to the name of the cells.

Of the SEQ ID NOs used in the specification, SEQ ID NO: 1 to SEQ ID NO: 9 designate the amino acid sequences of the following peptides.
[SEQ ID NO: 1] β-amyloid (1-38)
[SEQ ID NO: 2] β-amyloid (1-39)
[SEQ ID NO: 3] β-amyloid (1-40)
[SEQ ID NO: 4] β-amyloid (1-41)
[SEQ ID NO: 5] β-amyloid (1-42)
[SEQ ID NO: 6] β-amyloid (1-43)
[SEQ ID NO: 7] β-amyloid (1-28)
[SEQ ID NO: 8] β-amyloid (25-35)
[SEQ ID NO: 9] β-amyloid (35-43)

Throughout the specification, the proteins (polypeptides) are represented in accordance with the conventional way of describing peptides, that is, the N-terminus (amino terminus) at the left hand and the C-terminus (carboxyl terminus) at the right hand. In the proteins used in the present invention including the polypeptide having the amino acid sequence represented by SEQ ID NO: 1, the C-terminus may be in any form of a carboxyl group, a carboxylate, an amide and an ester.

As the β-amyloids in the present invention, there are employed β-amyloid (1-38) having the amino acid sequence represented by SEQ ID NO: 1, β-amyloid (1-39) having the amino acid sequence represented by SEQ ID NO: 2, β-amyloid (1-40) having the amino acid sequence represented by SEQ ID NO: 3, β-amyloid (1-41) having the amino acid sequence represented by SEQ ID NO: 4, β-amyloid (1-42) having the amino acid sequence represented by SEQ ID NO: 5, β-amyloid (1-43) having the amino acid sequence represented by SEQ ID NO: 6, etc. (preferably β-amyloid (1-42) having the amino acid sequence represented by SEQ ID NO: 5, etc.).

The derivatives of the β-amyloids used in the present invention include peptides each lacking about 1 to 17 amino acid residues from the N-terminal regions of the β-amyloids described above, peptides in which L-aspartic acid is isomerized to L-isoaspartic acid, D-isoaspartic acid or D-aspartic acid, peptides having pyroglutamic acid in the N-terminal regions, peptides having the amino acid sequences in which the 1 st to the 10th amino acids from the N-terminal regions in the β-amyloids described above are lacking and the N-terminal glutamic acid is converted into pyroglutamic acid, etc. Specific examples of the peptides used include a peptide having the 2nd to 42nd amino acid sequence in the amino acid sequence represented by SEQ ID NO: 5; a peptide having the 3rd to 42nd amino acid sequence in the amino acid sequence represented by SEQ ID NO: 5, in which the N-terminal glutamic acid is converted into pyroglutamic acid; a peptide having the 4th to the 42nd amino acid sequence in the amino acid sequence represented by SEQ ID NO: 5; a peptide lacking the 1st to the 16th amino acid sequence or the 1st to the 17th amino acid sequence in each of the amino acid sequences represented by SEQ ID NO: 1 through SEQ ID NO: 6 (e.g., β-amyloid (17-40), β-amyloid (18-40), etc.) and the like. These β-amyloids or derivatives thereof can be prepared, for example, from mammals such as humans, monkeys, rats, mice, etc. by publicly known methods, and may also be naturally occurring and purified samples which are commercially available. Alternatively, synthetic peptides may also be used.

In the present invention, examples of the partial peptides at the C-terminal region of the β-amyloids or derivatives thereof include the partial peptides having the amino acid sequences each beginning from the 25th amino acid from the N-terminal amino acid of the β-amyloids, etc.

As the β-amyloids or their derivatives described above, salts with physiologically acceptable acids (e.g., inorganic acids or organic acids), bases (e.g., alkali metal salts), etc. may be employed (preferably in the form of physiologically acceptable acid addition salts). Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Examples of the monoclonal antibodies in the present invention (hereinafter sometimes merely referred to as the antibodies), which specifically react with the partial peptides at the C-terminal region of the β-amyloids or derivatives thereof, include the antibodies which recognize the β-amyloids or derivatives thereof but do not recognize the partial peptide having the amino acid sequence represented by SEQ ID NO: 7 (i.e., the partial peptide at the N-terminal region of the β-amyloids, which is represented by β-amyloid (1-28)), and the like. Further examples include the antibodies which recognize the β-amyloids or derivatives thereof but do not recognize the partial peptide having the amino acid sequence represented by SEQ ID NO: 8 (i.e., the partial peptide at the mid-regions of the β-amyloids, which is represented by β-amyloid (25-35)); the antibodies which recognize the β-amyloids or derivatives thereof but do not recognize the partial peptide having the amino acid sequence represented by SEQ ID NO: 9 (i.e., the partial peptide at the C-terminal region of the β-amyloids, which is represented by β-amyloid (35-43)); and the like. More specifically, of these antibodies, the following antibodies are preferred:
(i) the antibodies which do not recognize the partial peptides each having the amino acid sequences represented by SEQ ID NO: 8 and SEQ ID NO: 9 (i.e., β-amyloid (25-35) and β-amyloid (35-43));
(ii) the antibodies which do not recognize the partial peptide having the amino acid sequences represented by SEQ ID NO: 8 (i.e., β-amyloid (25-35)), but recognize the partial peptide having the amino acid sequences represented by SEQ ID NO: 9 (i.e., β-amyloid (35-43)), and the like.

Of the se antibodies of (i) described above, preferred are the antibodies which particularly recognize β-amyloid (1-38) having the amino acid sequence represented by SEQ ID NO: 1, β-amyloid (1-39) having the amino acid sequence represented by SEQ ID NO: 2 and/or β-amyloid (1-40) having the amino acid sequence represented by SEQ ID NO: 3. Furthermore, preferred are the antibodies which recognize β-amyloid (1-38) having the amino acid sequence represented by SEQ ID NO: 1, β-amyloid (1-39) having the amino acid sequence represented by SEQ ID NO: 2, β-amyloid (1-40) having the amino acid sequence represented by SEQ ID NO: 3 but do not recognize β-amyloid (1-42) having the amino acid sequence represented by SEQ ID NO: 5.

Of the se antibodies of (ii) described above, preferred are the antibodies which particularly recognize the β-amyloids contained in the brain extracts with formic acid from patients with Alzheimer's disease (especially β-amyloid (1-42) having the amino acid sequence represented by SEQ ID NO: 5). Further preferred are the antibodies which recognize β-amyloid (1-42) represented by SEQ ID NO: 5 but do not recognize β-amyloid (1-38) having the amino acid sequence represented by SEQ ID NO: 1, β-amyloid (1-39) having the amino acid sequence represented by SEQ ID NO: 2 and β-amyloid (1-40) having the amino acid sequence represented by SEQ ID NO: 3.

A typical example of the antibodies of (i) described above is the monoclonal antibody indicated by BA-27a (see WO 94/17197), and typical examples of the antibodies of (ii) described above are the monoclonal antibodies indicated by BC-05a, BC-15a, BC-65a, BC-75a and BC-55a (particularly, BC-05a is preferred) (see WO 94/17197).

For methods of preparing antigens for the antibodies and methods of preparing the antibodies, publicly known methods such as the method described in WO 94/17197 or its modification can be used and are explained below by way of an example.

### (1) Preparation of Antigens

As antigens used for preparing the antibodies of the present invention, for example, any of the β-amyloids or derivatives thereof, partial peptides obtained by hydrolyzing the β-amyloids or derivatives thereof, and synthetic peptides having one or more antigenic determinants which are the same as those of the β-amyloids can be used (hereinafter sometimes briefly referred to as β-amyloid antigens). As the β-amyloids or derivatives thereof, those described above are used. These β-amyloids or derivatives thereof can be prepared, for example, from mammals such as humans, monkeys, rats, mice, etc. by publicly known methods or with some modifications, and may also be naturally occurring and purified samples which are commercially available. Alternatively, synthetic peptides may be used as well.

The β-amyloids described above or their derivatives or salts thereof can be produced by publicly known methods, for example, by the method described in WO 02/06483. They may also be (a) prepared from mammalian tissues or cells of human, monkey, rat, mouse, etc. by publicly known methods or with some modifications, (b) chemically synthesized by publicly known peptide synthesis methods using a peptide synthesizer, etc., or (c) produced by culturing transformants bearing DNAs encoding polypeptides comprising desired amino acid sequences or salts thereof.
(a) Where the β-amyloid antigen is prepared from the mammalian tissues or cells, the tissues or cells are homogenized and then extracted with an acid, an alcohol, etc., and the extract is purified and isolated by a combination of salting-out, dialysis, gel filtration, chromatography techniques such as reverse phase chromatography, ion exchange chromatography, affinity chromatography and the like.
(b) When the β-amyloid antigen is chemically synthesized, the synthetic peptide includes, for example, a peptide having the same structure as that of the β-amyloid antigen purified from the naturally occurring ones described above, etc.
(c) Where a polypeptide comprising the desired amino acid sequence or its salt is produced using a DNA-bearing transformant, the DNA can be produced in accordance with publicly known cloning techniques [e.g., the method described in Molecular Cloning (2nd ed., J. Sambrook et al., Cold Spring Harbor Lab. Press, 1989), etc.]. The cloning techniques include (1) a method in which a transformant bearing DNA encoding the polypeptide comprising the desired amino acid sequence or its salt is obtained from cDNA library by hybridization using a DNA probe or DNA primer designed based on the amino acid sequence of the polypeptide comprising the desired amino acid sequence or its salt, or (2) a method in which a transformant containing DNA encoding the polypeptide comprising the desired amino acid sequence or its salt is obtained by PCR using a DNA primer designed based on the amino acid sequence of the polypeptide comprising the desired amino acid sequence or its salt, etc.

Examples of the partial peptides obtained by hydrolyzing the β-amyloids include partial peptides obtained by hydrolyzing β-amyloid (1-43) having the amino acid sequence represented by SEQ ID NO: 6 successively from the N-terminus and/or the C-terminus with exoproteases such as aminopeptidase and carboxypeptidase or mixtures thereof, partial peptides obtained by hydrolyzing β-amyloid (1-43) with various endopeptidases or mixtures thereof; and the like. When β-amyloid (1-42) is prepared by this method, the resulting sample may occasionally be contaminated with β-amyloid (1-41) and/or β-amyloid (1-43) in some cases. Examples of the synthetic peptides include peptides having the same structure as the purified naturally occurring β-amyloid antigens described above, peptides having one or more amino acid sequences which are the same as those of any portions consisting of at least 3 amino acids, preferably at least 6 amino acids in the amino acid sequences of β-amyloid (1-43), etc. (hereinafter briefly referred to as β-amyloid-relating synthetic peptides).

The synthetic peptides described above can be produced according to public known conventional methods, which may be either solid phase synthesis or liquid phase synthesis. That is, the partial peptides or amino acids that can construct the peptides are condensed with the remaining part. Where the product contains protecting groups, these protecting groups are removed to give the desired peptide. Specific examples of such condensation methods or methods for removing protecting groups include methods described in B. Merrifield [J. Am. Chem. Soc., 85, 2149 (1963)], M. Bodanszky and M. A. Ondetti [Peptide Synthesis, Interscience Publishers, New York (1966)], Schroder and Lubke [The Peptide, Academic Press, New York, (1965)], N. Izumiya et al. [Peptide Gosei no Kiso to Jikken (Fundamentals and Experiments of Peptide Synthesis), Maruzen (1985)], H. Yazima and S. Sakakibara [Seikagaku Jikken Koza 1 (Course of Biochemical Experiments 1), Chemistry of Proteins IV, 205 (1977)], etc. For example, when the β-amyloids or the β-amyloid-relating synthetic peptides are synthesized by the solid phase process, any resins known in the art as insoluble resins (such as chloromethyl resins, 4-oxymethylphenylacetamidomethyl resins, etc.) are used for a successive condensation of protected amino acids in a conventional manner from the C-terminal sides of the β-amyloids or the β-amyloid-relating synthetic peptides. Then, all the protective groups are removed by a hydrogen fluoride treatment, followed by purification by publicly known methods, such as high performance liquid chromatography, etc. Thus, the desired β-amyloids or β-amyloid-relating synthetic peptides can be obtained. The N-protected amino acids can be produced by such methods that an α-amino group is protected with Boc group; the hydroxyl group of, e.g., serine and threonine is protected with Bzl group; the ω-carboxyl group of glutamic acid or aspartic acid is protected with OBzl group; the e-amino group of lysine is protected with Cl-Z group; the hydroxyl group of tyrosine is protected with Br-Z group; the guanido group of arginine is protected with Tos group; and the imidazole group of histidine is protected with Bom group. After the reaction, the product may be purified and isolated by a combination of conventional purification methods such as solvent extraction, distillation, column chromatography, liquid chromatography, recrystallization, etc. to give the peptide. When the peptide obtained by the above methods is in a free form, the peptide can be converted into an appropriate salt by a publicly known method; conversely when the peptide is obtained in a salt form, it can be converted into a free form by a publicly known method.

The amides of the peptide may be obtained using commercially available resins for peptide synthesis, which are suitable for formation of the amides. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin,
4- (2',4'- dimethoxyphenylhydroxymethyl)phenoxy resin,
4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective peptide according to various condensation methods publicly known in the art. At the end of the reaction, the peptide is cut out from the resin and at the same time, the protecting groups are removed to obtain the objective peptide.
Alternatively, the objective peptide may also be obtained by using chlorotrityl resin, oxime resin, 4-hydroxybenzoic acid type resin, etc., taking out the peptide protected in part, and removing the protective groups from the peptide in a conventional manner.

For condensation of the protected amino acids described above, a variety of activation reagents for peptide synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the amino acids previously protected in the form of symmetric acid anhydrides, HOBt esters or HOOBt esters are activated, followed by adding the thus activated protected amino acids to the resin. Solvents suitable for use to activate the protected amino acids or condense with the resin may be appropriately chosen from solvents known to be usable for peptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; tertiary amines such as pyridine, etc.; ethers such as dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to peptide bond-forming reactions and is usually selected in the range of approximately -20°C to 50°C. The activated amino acid derivatives are used generally in an excess of about 1.5 to about 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel adverse effects on the subsequent reactions.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting groups of a carboxyl group include a C₁₋₆ alkyl group, a C₃₋₈ cycloalkyl group and a C₇₋₁₄ aralkyl group, 2-adamantyl, 4-nitrobenzyl, 4-methoxybenzyl, 4-chlorobenzyl, phenacyl and benzyloxycarbonyl hydrazide, t-butoxycarbonyl hydrazide, trityl hydrazide and the like.

The hydroxyl group of serine and threonine can be protected through, for example, its esterification or etherification. Examples of the groups appropriately used for the esterification include a lower (C₁₋₆) alkanoyl group, such as acetyl group, etc.; an aroyl group such as benzoyl group, etc., and a group derived from carbonic acid such as benzyloxycarbonyl group, ethoxycarbonyl group, etc. Examples of a group suitable for the etherification include benzyl group, tetrahydropyranyl group, t-butyl group, etc.

Examples of the groups for protecting the phenolic hydroxyl group of tyrosine include Bzl, Cl-Bz1, 2-nitrobenzyl, Br-Z, t-butyl, etc.

Examples of the groups used to protect the imidazole moiety of histidine include Tos, 4-methoxy-2,3,6-trimethylbenzenesulfonyl, DNP, Bom, Bum, Boc, Trt, Fmoc, etc.

Examples of the activated carboxyl groups in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. Example of the activated amino groups in the starting materials include the corresponding phosphoric amides.

To eliminate (split off) the protecting groups, there are used catalytic reduction under hydrogen gas flow in the presence of a catalyst such as Pd-black, Pd-carbon, etc.; an acid treatment with anhydrous hydrofluoric acid, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia; or the like. The elimination of the protecting groups by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, removal of the protecting groups and activation of the functional groups involved in the reaction may be appropriately chosen from publicly known groups and publicly known means.

In another method for obtaining the amides of the peptide, for example, the α-carboxyl group in the carboxyl terminal amino acid is first protected by amidation; the peptide chain is then extended to a desired length toward the amino group side. Thereafter, a peptide in which only the protecting group of the N-terminal α-amino group in the peptide chain has been removed from the peptide and a peptide (or amino acids) in which only the protecting group of the C-terminal carboxyl group has been removed are prepared. The two peptides are condensed in a mixture of the solvents described above. The details of the condensation reaction are the same as described above. After the protected peptide obtained by the condensation is purified, all the protecting groups are removed by the methods described above to give the desired crude peptide. This crude peptide is purified by various known purification means. Lyophilization of the major fraction gives the amides of the desired peptide.

To prepare the esterified peptide, for example, the α-carboxyl group of the carboxy terminal amino acid is condensed with a desired alcohol to prepare the amino acid ester, which is followed by a procedure similar to the preparation of the amidated peptide above to give the ester form of the desired peptide.

The β-amyloid antigen can aggregate easily and thus insolubilized antigen can also be directly immunized. Furthermore, a complex in which the β-amyloid antigen is bound or adsorbed to an appropriate carrier may also be used for immunization. For the carrier and the mixing ratio of the carrier to the β-amyloid antigen (hapten), the antigen may be bound or adsorbed to any carrier in any ratio, as long as an antibody is effectively raised to the β-amyloid antigen bound or adsorbed to the carrier. A complex can be used in which the hapten antigen is bound or adsorbed to a natural or synthetic polymer carrier, which is usually used in preparing an antibody to the hapten antigen in a weight ratio of 0.1 to 100 based on one hapten. The natural polymer carrier includes, for example, serum albumin of a mammal such as bovine, rabbit, human, etc., thyroglobulin of a mammal such as bovine, rabbit, etc., hemoglobin of a mammal such as bovine, rabbit, human, sheep, etc., and keyhole limpet hemocyanin. Examples of the synthetic high molecular carrier, which can be used, are various latexes including a polymers, copolymers, etc., for example, polyamino acids, polystyrenes, polyacryls, polyvinyls, polypropylenes, etc.

For coupling of the hapten and the carrier, a variety of condensing agents can be used. Examples of the condensing agents, which are advantageously employed, are diazonium compounds such as bis-diazotized benzidine capable of crosslinking tyrosine, histidine or tryptophan; dialdehyde compounds such as glutaraldehyde, etc. capable of crosslinking amino groups with each other; diisocyanate compounds such as toluene-2,4-diisocyanate, etc.; dimaleimide compounds such as N,N'-o-phenylenedimaleimide, etc. capable of crosslinking thiols with each other; maleimide activated ester compounds capable of crosslinking an amino group with a thiol group; carbodiimide compounds capable of crosslinking an amino group with a carboxyl group; etc. In the crosslinking of amino groups with each other, one amino group is reacted with an activated ester reagent (e.g., SPDP, etc.) having dithiopyridyl group and then reduced to introduce the thiol group, whereas a maleimide group is introduced into another amino group using a maleimide activated ester reagent, and the two groups may be reacted with each other.

### (2) Preparation of monoclonal antibody

The β-amyloid antigen is administered to a warm-blooded animal either solely or toge ther with a carrier or diluent to the site where antibody production is possible by administration routes such as intraperitoneally, intravenously, subcutaneously, etc. In order to potentiate the antibody productivity upon the administration, complete Freund's adjuvants or incomplete Freund's adjuvants may be administered. The administration is usually carried out once in every 2 to 6 weeks and approximately 2 to 10 times in total. Examples of the warm-blooded animal are monkey, rabbit, dog, guinea pig, mouse, rat, sheep, goat, fowl, etc. with mouse and rat being preferred for production of monoclonal antibodies.

In the preparation of the monoclonal antibodies, from warm-blooded animals, e.g., mice, immunized with the β-amyloid antigen, the animal wherein the antibody titer is noted is selected, then the spleen or lymph node is collected after 2 to 5 days from the final immunization and antibody-producing cells contained therein are fused with myeloma cells to give anti-β-amyloid monoclonal antibody-producing hybridomas. Measurement of the anti-β-amyloid antibody titer in sera is made, for example, by reacting a labeled form of the β-amyloid, which will be described later, with the antiserum followed by measuring activity of the labeling agent bound to the antibody. The fusion may be operated, for example, by the known Kohler and Milstein method [Nature, 256, 495 (1975)]. Examples of fusion accelerators are polyethylene glycol (PEG), Sendai virus, etc., of which PEG is preferably employed. Examples of the myeloma cells are NS-1, P3U1, SP2/0, AP-1, etc. In particular, P3U1 or the like is preferably employed. A preferred ratio in count of the antibody-producing cells (spleen cells) to the myeloma cells used is within a range of approximately 1:1 to 20:1. When PEG (preferably, PEG 1000 to PEG 6000) is added in a concentration of approximately 10 to 80% followed by incubation generally at 20 to 40°C, preferably at 30 to 37°C generally for 1 to 10 minutes, an efficient cell fusion can be carried out.

Various methods can be used for screening of the anti-β-amyloid antibody-producing hybridomas. Examples of such methods include a method which comprises adding the hybridoma supernatant to a solid phase (e.g., microplate) adsorbed with the β-amyloid or β-amyloid-relating synthetic peptide directly or together with a carrier, then adding an anti- immunoglobulin antibody (when mouse cells are used for the cell fusion, anti- mouse immunoglobulin antibody is used) labeled with a radioactive substance, an enzyme or the like, or Protein A and detecting the anti-β-amyloid monoclonal antibody bound to the solid phase; a method which comprises adding the hybridoma supernatant to a solid phase adsorbed with an anti-immunoglobulin antibody or Protein A, adding the β-amyloid labeled with a radioactive substance, an enzyme, etc. and detecting the β-amyloid monoclonal antibody bound to the solid phase; etc. Screening and plating of the anti-β-amyloid monoclonal antibody can be performed generally in a medium for animal cells (e.g., RPMI 1640) containing 10-20% fetal calf serum and supplemented with HAT (hypoxanthine, aminopterin and thymidine). The antibody titer in the hybridomas culture supernatant can be assayed as in the assay for the anti-β-amyloid antibody titer in the antisera described above.

Separation and purification of the anti-β-amyloid monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which involves collecting only an antibody with an activated adsorbent such as a antigen-binding solid phase, Protein A, Protein G, KAPTIV-AE (TECNOGEN S.C.P.A.), etc. and dissociating the binding to obtain the antibody; and the like].

Furthermore, the hybridoma producing the anti-β-amyloid antibody reactive with a partial region of the β-amyloid and the hybridoma producing the anti-β-amyloid monoclonal antibody reactive with the β-amyloid, but not reactive with its partial region can be selected, for example, by measuring the binding property of a peptide corresponding to the partial region and an antibody produced by the hybridoma.

As described above, the antibody of the present invention can be produced by culturing hybridoma cells in a warm-blooded animal in vivo or in vitro and collecting the antibody from the body fluids or culture medium.

The anti-β-amyloid monoclonal antibody thus obtained may be used as the agent for preventing/treating (including a progression-retarding agent) Alzheimer's disease, mild cognitive impairment (MCI), cerebral amyloid angiopathy (CAA), etc. (preferably, Alzheimer's disease, etc). Furthermore, the preventing/treating agent is preferably an agent for suppressing aggregation or deposition of the β-amyloid in the brain, or an agent for specifically increasing the blood level of a peptide having the amino acid sequences represented by SEQ ID NO: 5, etc.

For these purposes, the antibody molecule itself may be used, or F(ab')₂, Fab' or Fab fractions of the antibody molecule may be used. For the antibody of the present invention used for these purposes, antibodies which pass through the blood brain barrier (BBB) are preferred. Of these antibodies used for these purposes, preferred are an antibody which prevents deposition of the β-amyloid, an antibody which inhibits oligomer formation of the β-amyloid, or an antibody which is capable of drawing the β-amyloid out of the senile plaques formed (or an antibody which is capable of inducing disappearance of the senile plaques formed in the brain), and the like. Alternatively, the antibody may be an antibody which does not pass through the blood-brain barrier (BBB); in this case, the antibody is preferably capable of capturing the peripheral β-amyloid in the periphery to promote discharge of the β-amyloid in the brain into the periphery.

The antibody which is capable of passing through the blood-brain barrier to bind to the β-amyloid in the senile plaques formed can also be used as a direct *in vivo* diagnostic composition for Alzheimer's disease, mild cognitive impairment (MCI), cerebral amyloid angiopathy (CAA), etc. It is known that diffuse senile plaque s seen in Alzheimer's disease at very early stages or mild cognitive impairment (MCI) are mainly composed of β-amyloid (x-42). In particular, by imaging a direct *in vivo* binding of the antibody specific to the C-terminal region of β-amyloid (x-42) or the F(ab')₂, Fab' or Fab fraction of the antibody molecule, etc. to the diffuse senile plaques using high field MRI or the like, early diagnosis of Alzheimer's disease or mild cognitive impairment (MCI) can be facilitated.

An agent for preventing/treating Alzheimer's disease, mild cognitive impairment (MCI), cerebral amyloid angiopathy (CAA), etc., (preferably, Alzheimer's disease, etc), comprising the antibody of the present invention is safe and low toxic, and can be administered parenterally or orally to human or mammals (e.g., rats, rabbits, sheep, swine, bovine, cats, dogs, monkeys, etc.) as it is in the form of liquid preparations or as a pharmaceutical composition of appropriate dosage form. The dose may vary depending upon subject to be administered, target disease, conditions, route of administration, etc. For example, when the agent is used for the purpose of treating, e.g., Alzheimer's disease in an adult, it is advantageous to administer the antibody of the present invention parenterally generally in a single dose of approximately 0.01 to 20 mg/kg body weight, preferably approximately 0.1 to 10 mg/kg body weight, and more preferably approximately 0.1 to 5 mg/kg body weight, approximately 1 to 5 times per week, preferably approximately 1 to 4 times per month. In oral administration, the agent can be administered in a dose corresponding to the dose given above. When the condition is especially severe, the dose may be increased according to the condition.

The antibody of the present invention may be administered in itself or as an appropriate pharmaceutical composition. The pharmaceutical composition used for the administration described above comprises the antibody described above or its salt and a pharmacologically acceptable carrier, a diluent or an excipient. Such a pharmaceutical composition is provided in a dosage form suitable for oral or parenteral administration.

Examples of the composition for oral administration include a solid or liquid dosage form, more specifically, tablets (including dragees and film-coated tablets), pills, granules, powders, capsules (including soft capsules), syrups, emulsions, suspensions, etc. Such a composition is manufactured by publicly known methods and comprises carriers, diluents or excipients conventionally used in the field of pharmaceutical preparations. As the carriers and excipients for tablets e.g., lactose, starch, sucrose and magnesium stearate are used.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by publicly known methods. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate dissolution aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a dissolution aid such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Advantageously, the pharmaceutical compositions for parenteral or oral use described above are prepared into pharmaceutical preparations with a unit dose suited to fit a dose of the active ingredients. Such unit dose preparations include, for example, tablets, pills, capsules, injections (ampoules) and suppositories. The amount of the antibody contained is generally about 5 to about 500 mg per dosage unit form; it is preferred that the aforesaid antibody is contained in about 5 to about 100 mg especially in the form of injection, and in about 10 to 250 mg for the other forms.

Each of the compositions described above may further contain other active ingredients, unless any adverse interaction occurs due to blending with the antibody described above.

In the specification of the present invention, when amino acids, etc. are shown by abbreviations and in this case, they are denoted in accordance with the IUPAC-IUB Commission on Biochemical Nomenclature or by the common codes in the art, examples of which are listed below. For amino acids that may have the optical isomer, L form is presented unless otherwise indicated.
PAM : phenylacetamidomethyl
Boc : t-butyloxycarbonyl
Z : benzyloxycarbonyl
Cl-Z 2-chlorobenzyloxycarbonyl
Br-Z :2-bromobenzyloxycarbonyl
Bzl : benzyl
OcHex : cyclohexyl ester
OBzl : benzyl ester
Tos :p-toluenesulfonyl
HOBt : 1-benzotriazole
MeBzl : 4- methylbenzyl
Bom : benzyloxymethyl
DCC : N,N'-dichlorohexylcarbodiimide
TFA : trifluoroacetic acid
DMF : N,N-dimethylformamide
Gly : glycine
Ala : alanine
Val : valine
Leu :leucine
Ile : isoleucine
Ser : serine
Thr :threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gln glutamine
SPDP : N-succinimidyl 3-(2-pyridyldithio)propionate
GMBS : N-(4-maleimidobutyryloxy)succinimide
BSA : bovine serum albumin
BTG : bovine thyroglobulin
EIA : enzyme immunoassay
HPLC : reversed phase high performance liquid chromatography
HRP : horse radish peroxidase
FBS : fetal bovine serum
d-FBS : dialyzed fetal bovine serum
TMB :3,3',5,5'-tetramethylbenzidine
H/HBSS: HEPES buffered Hanks' balanced solution

The sequence identification numbers used in the present specification represent the amino acid sequences of the following peptides.
[SEQ ID NO:1] β-amyloid (1-38)
[SEQ ID NO: 2] β-amyloid (1-39)
[SEQ ID NO: 3] β-amyloid (1-40)
[SEQ ID NO: 4] β-amyloid (1-41)
[SEQ ID NO: 5] β-amyloid (1-42)
[SEQ ID NO: 6] β-amyloid (1-43)
[SEQ ID NO: 7] β-amyloid (1-28)
[SEQ ID NO: 8] β-amyloid (25-35)
[SEQ ID NO: 9] β-amyloid (35-43)

Hereinafter, the present invention will be described in more detail with reference to EXAMPLES but they are not deemed to limit the scope of the invention.

Monoclonal antibodies BC-05a and BA-27a were obtained in accordance with the procedures described in EXAMPLE 7 of WO 94/17197.

### EXAMPLE 1

### (1) Assay of Aβx-40 and Aβx-42 by the sandwich assay-EIA

BALB/C mice were immunized with β-amyloid (11-28) by a modification of the procedures described in EXAMPLE 7 of WO 94/17197 to obtain monoclonal antibody BNT-77a (Asami-Odaka, A. et al., Biochemistry, 34, 10272-10278, 1995). A 100 µl aliquot of 0.1 M carbonate buffer (pH 9.6) solution containing 15 µg/ml of BNT-77a was dispensed in a 96-well microplate, which was then allowed to stand at 4 °C for 24 hours. Subsequently, 300 µl of Block Ace (Dainippon Pharmaceutical) diluted to 4- fold with PBS was added to inactivate the excess binding sites of the wells. For assay of Aβx-40, the serial dilution of Aβ1-40 (Peptide Institute, Inc.) with buffer EC [0.02 M phosphate buffer containing 10% Block Ace, 0.2% BSA, 0.4 M NaCl, 0.05% CHAPS, 2 mM EDTA and 0.05% NaN₃, pH 7] and 100 µl of a test fluid were added to the primary antibody-immobilized plate as described above, followed by reaction at 4 °C for 24 hours. After washing with PBS, 100 µl of BA-27a-HRP (cf. WO 94/17197), as a secondary antibody, diluted to 1000-fold with buffer C [0.02 M phosphate buffer containing 1% BSA, 0.4 M NaCl and 2 mM EDTA, pH 7] was added to the reaction mixture, followed by reaction at room temperature for 6 hours. After the plate was washed with PBS, 100 µl of a TMB microwell peroxidase substrate system (KIRKEGAARD & PERRY LAB, INC.) was added to the reaction mixture, followed by reaction at room temperature for 10 minutes. After the reaction was terminated by adding 100 µl of 1M phosphoric acid, the enzyme activity on the solid phase was determined by measuring the absorbance at 450 nm on a plate reader (SPECTRAMAX 190, Molecular Device, Inc.). The Aβx-42 was assayed in a manner similar to the Aβx-40 by adding the serial dilution of Aβ1-42 (Peptide Institute, Inc.) and a test fluid to the BNT-77a-immobilized plate prepared above and using BC-05a-HRP (cf. WO 94/17197) as a secondary antibody.

### (2) Change in the levels of Aβx-40 and Aβx-42 in the plasma and in the cerebrospinal fluid of BALB/C mice after intraperitoneal administration of BA-27a or BC-05a

The effects of the antibodies above were examined in 19 BALB/C mice of 3 months old. BA-27a, 0.5 mg, was intraperitoneally given to 7 mice and 0.5 mg of BC-05a to 6 mice, respectively. The remaining 6 mice, which received none, were examined as control. Under ether anesthesia, the plasma and the cerebrospinal fluid (CSF) were collected from mice 16 hours after the administration, and the Aβx-40 and Aβx-42 were assayed. In addition, the plasma was collected 4 times every other week from mice intraperitoneally given with BC-05a, and the Aβx-42 was assayed. After 10 µl of the plasma was reacted with 5 µg of Dynabeads M280 anti- mouse Ig (Dynal) for 2 hours at room temperature, the supernatant was recovered. The immunoglobulin- unbound Aβx-40 and Aβx-42 in the plasma and the immunoglobulin-bound Aβx-40 and Aβx-42, which were eluted with 0.2 M glycine at pH 2.8, were assayed.

The Aβx-40 in the plasma was found to be 137.3 ± 2.2 fmol/ml in the group administered with BA-27a, 3.3 ±1.2 fmol/ml in the group administered with BC-05a and 3.4 ± 1.8 fmol/ml in the normal control group.

The Aβx-40 in the cerebrospinal fluid was found to be 670.8 ± 252.6 fmol/ml in the group administered with BA-27a, 166.5 ± 140.1 fmol/ml in the group administered with BC-05a and 246 ± 184.3 fmol/ml in the normal control group. The Aβx-42 in the plasma was found to be 0.5± 0.4 fmol/ml in the group administered with BA-27a, 60.7± 7.1 fmol/ml in the group administered with BC-05a and 0.2 ± 0.2 fmol/ml in the normal control group.

The Aβx-42 in the cerebrospinal fluid was less than the measurement sensitivity in the group administered with BA-27a, 0.6 ± 0.4 fmol/ml in the group administered with BC-05a and less than the measurement sensitivity in the normal control group.

When BA-27a was administered, the Aβx-40 was significantly increased by 40 times in the plasma and by 2.7 times in the cerebrospinal fluid (p<0.001). Any increase of the Aβx-40 was not observed either in the plasma or in the cerebrospinal fluid when BC-05a was administered.

No increase of the Aβx-42 was observed either in the plasma or in the cerebrospinal fluid when BA-27a was administered. When BC-05a was administered, the Aβx-42 was significantly increased by 304 times in the plasma (p<0.001) and increased to 0.6 ± 0.4 fmol/ml in the cerebrospinal fluid, though the Aβx-42 was less than the measurement sensitivity in the normal control group.

In mice continuously administered with BC-05a for consecutive one month, only plasma Aβx-42 was increased to 324.1 fmol/ml or by 1621 times. Furthermore, differentiation of the antibody-bound type and the antibody unbound type by immunoprecipitation revealed that almost 100% of the increased Aβx-42 was of the antibody unbound type.

The foregoing results demonstrate that when anti Aβ42C terminus-specific antibody BC-05a was intraperitoneally given to mice, the Aβx-42 in the cerebrospinal fluid and the Aβx-42 in the plasma were markedly increased specifically. Since the Aβx-42 increased in the plasma was of the immunoglobulin-unbound type, this increase of Aβx-42 was not the measurement of the Aβx-42, to which the BC-05a given was bound in blood.

It is therefore considered that the administered BC-05a resulted in increasing the cerebral clearance of Aβx-42 thereby to increase the Aβx-42 in the cerebrospinal fluid and the Aβx-42 in the plasma.

The results show that by administering BC-05a, the brain level of Aβx-42 as an onset factor of Alzheimer's disease can be selectively controlled and furthermore, the deposition of cerebral amyloid Aβx-42, which once deposited to cause various pathological processes, can be selectively removed and ameliorated. By applying the results that the administered BC-05a binds to the Aβx-42 in the brain, it is considered applicable also to a new diagnostic strategy including selective imaging of senile plaques, selective estimation of the level of amyloid Aβx-42 deposited in the brain by intravascular administration of BC-05a, etc.

### EXAMPLE 2

### (1) Preparation of biotinylated BC-05a and biotinylated mouse IgG

After 40 µl of sulfo-NHS-biotin (Pierce, Inc.) aqueous solution (2.4 mg/120 µl) was added to 2 ml of BC-05a (10 mg/ml), the mixture was reacted at room temperature for 30 minutes while stirring. The reaction solution was diluted, followed by dialysis to PBS (-) at 4°C for 2 days. Thereafter the antibody concentration was determined. The yield was about 70%. Mouse IgG (Wako Pure Chemicals) used for control was biotinylated as well.

### (2) Continuous administration of the biotinylated antibody to young APPswTg (Tg2576) mice for short term, preparation of brain soluble fraction and determination of antibody concentration in the brain

The biotinylated mouse IgG or biotinylated BC-05a (0.5 mg each/0.2 ml/mouse) was administered in a bolus intraperitoneally to female Tg2576 mice (12 weeks old) (Science, 274, 99-102, 1996) and blood was collected 24 hours later. After infusion, the brain was taken, cut in half and frozen (n = 4-5). Protease Inhibitor Cocktail (Complete, Roche) and EDTA (final concentration of 4 mg/ml) were added to and mixed with the blood. After centrifugation, the supernatant was freeze- stored as plasma. A 4- fold amount by weight of 50 mM Tris-hydrochloride-buffered saline solution (pH 7.5) (TS buffer) containing Protease Inhibitor Cocktail was added to the cerebral hemisphere and homogenized, followed by centrifugation at 300,000 x g at 4°C for 20 minutes. The resulting supernatant was used as the brain soluble fraction. The fraction was added to an avidin- immobilized 96-well plate (Reacti-Bind NeutrAvidin Coated Plate, Pierce, Inc.), which was then reacted overnight at 4°C. After washing, anti-mouse IgG-HRP (Amersham, Inc.) was added, followed by reaction at room temperature for 6 hours. After washing, the substrate for HRP (TMB Microwell Peroxidase substrate system, KPL) was added. By adding 1M phosphoric acid, the reaction was terminated and the absorbance at 450 nm was then measured on a plate reader. The antibody concentration in the brain was calculated using attached calculation software (SoftMAX). The antibody concentration in the plasma was determined as well. A calibration curve obtained using BC-05a as the standard was prepared and the results of the concentrations of biotinylated BC-05a produced in (1) above in the plasma and in the brain are shown in TABLE 1.

**[TABLE 1]**

| | Biotinylated mouse IgG group | Biotinylated BC-05a group |
|---|---|---|
| | (n=4) (pmol/g wet tissue) | (n=5) (pmol/g wet tissue) |
| Plasma | 1300±296 | 1030±94.8 |
| Brain soluble fraction | 6.42±0.475 | 4.75±0.056 |

| | | |
|---|---|---|
| mean ± S.E.M. | | |

The results reveal that approximately 0.5% of the antibody concentration in the plasma was transported into the brain, even when any one of the biotinylated BC-05a and the biotinylated IgG was administered.

### EXAMPLE 3

### Continuous administration of BC-05a to young APPswTg (Tg2576) mice for consecutive 9 months, preparation of the brain extract and assay of Aβ levels in the blood and in the brain

Mouse IgG or BC-05a (0.5 mg each/0.2 ml/mouse up to 15 weeks old, 1.0 mg each/0.2 ml/mouse on or after 16 weeks old) was intraperitoneally administered once a week to male Tg2576 mice (10 weeks old) (Science, 274, 99-102, 1996) until the animal reached 52 weeks old (12 months old) (n = 9-10). Sampling was performed in the same way as described above.

The soluble and insoluble fractions were separately prepared from the brain extract. The soluble fraction was prepared in the same way as in EXAMPLE 2. After ultracentrifugation, the precipitates were washed with TS buffer and a 8- fold amount of the brain hemisphere by weight of 50 mM Tris-hydrochloride aqueous solution (pH 7.5) containing 6M guanidine hydrochloride was added to dissolve. After centrifugation at 15000 rpm at 4°C for 20 minutes, the supernatant was used as the insoluble fraction. The Aβ level was determined following the procedures described in EXAMPLE 1. For the brain insoluble fraction, a 2000-fold dilution with buffer EC was used as a sample.

The Aβ level in the plasma was determined in two ways, namely, by the assay in which the diluted plasma was directly assayed and by the assay after dissociation of the BC-05a complex with guanidine hydrochloride followed by dilution. In the latter way, 30 µl of 50 mM Tris-hydrochloride aqueous solution (pH 7.5) containing 8M guanidine hydrochloride was added to and mixed with 10 µl of the plasma and then diluted by adding 560 µl of buffer EC to the mixture. The resulting dilution was used as the sample for ELISA.

The results are shown in TABLE 2.

**[TABLE 2]**

| | | Aβx-40 | Aβx-42 |
|---|---|---|---|
| | | (pmol/g wet tissue) | (pmol/g wet tissue) |
| Plasma (intact) | | | |
| | IgG Group | 3160 ± 302 | 389 ± 20.0 |
| | BC-05a Group | 2270 ± 186 | 8810 ± 224 |

| Plasma (treated with guanidine) | | | |
|---|---|---|---|
| | IgG Group | 2990 ± 208 | 588 ± 29.0 |
| | BC-05a Group | 2350 ± 128 | 18800 ± 1100 |

| Brain soluble Aβ | | | |
|---|---|---|---|
| | IgG Group | 2.23 ± 0.402 | 0.413 ± 0.0521 |
| | BC-05a Group | 2.29 ± 0.27 | 0.643 ± 0.0687 |

| Brain insoluble Aβ | | | |
|---|---|---|---|
| | IgG Group | 1650 ± 383 | 645 ± 102 |
| | BC-05a Group | 1200 ± 347 | 442 ± 57.4 |

| | | | |
|---|---|---|---|
| mean ± S.E.M. | | | |

As in the bolus administration, the plasma Aβ level was markedly increased from 22 times to 32 times. By treating the plasma with guanidine, the plasma Aβx-42 level in the BC-05a group was increased by approximately 2 times, suggesting that BC-05a would contribute to stabilization of the Aβx-42 in blood.

Any change in the soluble Aβx-40 level in the brain was not observed but a reducing tendency of the insoluble Aβx-40 level was observed. The Aβx-42 level in the brain was significantly increased for the soluble fraction but a reducing tendency for the insoluble fraction was observed.

### INDUSTRIAL APPLICABILITY

By the use of a monoclonal antibody, which specifically reacts with a partial peptide at the C-terminal region of a β-amyloid or its derivatives and does not recognize a partial peptide having the amino acid sequence represented by SEQ ID NO: 8, the β-amyloid having a C-terminal hydrophobic region can be specifically recognized. This antibody is useful as an agent for preventing/treating Alzheimer's disease, etc., or as a diagnostic agent for Alzheimer's disease, etc. In addition, although it is expected to be poorly efficient to transport β-amyloid (x-42) out of the brain via the blood-brain barrier because the ratio of β-amyloid (x-42) is less in the soluble fraction such as cerebrospinal fluid, etc., the antibody described above can selectively increase the blood level of β-amyloid (x-42) alone and reduce insoluble β-amyloid (x-42) in the brain. Moreover, the antibody described above is little reactive with the cerebrovascular amyloid mainly composed of β-amyloid (x-40) and thus considered not to induce cerebral hemorrhage including cerebral amyloid angiopathy.

## Claims

1. An agent for preventing/treating Alzheimer's disease, mild cognitive impairment or cerebral amyloid angiopathy, comprising a monoclonal antibody, which specifically reacts with a partial peptide at the C-terminal region of a β-amyloid or a derivative thereof and does not recognize a partial peptide having the amino acid sequence represented by SEQ ID NO: 8.

2. The agent according to claim 1, which is an agent for preventing/treating Alzheimer's disease.

3. The agent according to claim 1, wherein said antibody is an antibody which does not recognize a partial peptide having the amino acid sequence represented by SEQ ID NO: 9.

4. The agent according to claim 1, wherein said antibody is an antibody which recognizes a partial peptide having the amino acid sequence represented by SEQ ID NO: 9.

5. The agent according to claim 1, wherein said β-amyloid is a peptide having the amino acid sequence represented by SEQ ID NO: 1, SEQ ID NO: 2, SEQ ID NO: 3, SEQ ID NO: 4, SEQ ID NO: 5 or SEQ ID NO: 6.

6. The agent according to claim 1, wherein said β-amyloid is a peptide having the amino acid sequence represented by SEQ ID NO: 5.

7. The agent according to claim 1, wherein said derivative of the β-amyloid is a peptide having the amino acid sequence from the 2nd to the 42nd in the amino acid sequence represented by SEQ ID NO: 5, a peptide having the amino acid sequence from the 3rd to the 42nd in the amino acid sequence represented by SEQ ID NO: 5, in which the N-terminal glutamic acid is converted into pyroglutamic acid, or a peptide having the amino acid sequence from the 4th to the 42nd in the amino acid sequence represented by SEQ ID NO: 5.

8. The agent according to claim 1, wherein said derivative of the β-amyloid is a peptide having an amino acid sequence lacking the 1st to the 10th amino acid sequence in each of the amino acid sequences represented by SEQ ID NO: 1 through SEQ ID NO: 6, in which the N-terminal glutamic acid is converted into pyroglutamic acid.

9. The agent according to claim 1, wherein said partial peptide at the C-terminal region of the β-amyloid or a derivative thereof is a partial peptide having an amino acid sequence beginning from the 25th amino acid from the N-terminal amino acid of each β-amyloid.

10. The agent according to claim 1, wherein said antibody is an antibody which does not recognize a partial peptide having the amino acid sequence represented by SEQ ID NO: 7.

11. The agent according to claim 1, wherein said antibody is an antibody which recognizes β-amyloid (1-42) having the amino acid sequence represented by SEQ ID NO: 5.

12. The agent according to claim 1, wherein said antibody is monoclonal antibody BA-27a, which is producible from the hybridoma indicated byBA-27 (FERM BP-4139).

13. The agent according to claim 1, wherein said antibody is monoclonal antibody BC-05a, which is producible from the hybridoma indicated by BC-05 (FERM BP-4457).

14. The agent according to claim 1, wherein said antibody passes through a blood-brain barrier.

15. The agent according to claim 14, wherein said antibody is an antibody capable of drawing the β-amyloid out of the senile plaques formed.

16. The agent according to claim 1, which is an agent for suppressing aggregation or deposition of the β-amyloid in the brain.

17. The agent according to claim 1, which is capable of specifically increasing the blood level of a peptide having the amino acid sequence represented by SEQ ID NO: 5.

18. The agent according to claim 1, wherein said antibody is an antibody which does not pass through a blood-brain barrier.

19. The agent according to claim 18, wherein said antibody is an antibody capable of capturing the peripheral β-amyloid in the periphery.

20. A method for preventing/treating Alzheimer's disease, mild cognitive impairment or cerebral amyloid angiopathy, which comprises administering to a mammal an effective dose of a monoclonal antibody, which specifically reacts with a partial peptide at the C-terminal region of a β-amyloid or a derivative thereof and does not recognize a partial peptide having the amino acid sequence represented by SEQ ID NO: 8.

21. Use of a monoclonal antibody, which specifically reacts with a partial peptide at the C-terminal region of a β-amyloid or a derivative thereof and does not recognize a partial peptide having the amino acid sequence represented by SEQ ID NO: 8, to produce an agent for preventing/treating Alzheimer's disease, mild cognitive impairment or cerebral amyloid angiopathy.

22. The agent according to claim 1, wherein said antibody recognizes β-amyloid (1-42) having the amino acid sequence represented by SEQ ID NO: 5 but does not recognize any of β-amyloid (1-38) having the amino acid sequence represented by SEQ ID NO: 1, β-amyloid (1-39) having the amino acid sequence represented by SEQ ID NO: 2 and β-amyloid (1-40) having the amino acid sequence represented by SEQ ID NO: 3.


SEQUENCE LI STI NG

<110> Takada Phar maccut i cal Company Li mi t cd

<120> Use of ant i body

<130> G06- 0002

<140> PCT/ JP2004/ 013397
<141> 2004- 09- 08

<150> JP2003- 317443
<151> 2003- 09- 09

<160> 9

<210> 1
<211> 38
<212> PRT
<213> Homo sapi ens

<400> 1
Asp Al a Gl u Phc Arg Hi s Asp Scr Gl y Tyr Gl u Val Hi s Hi s Gl n Lys
5 10 15
Leu Val Phe Phe Al a Gl u Asp Val Gl y Ser Asn Lys Gl y Al a Il e Il e
20 25 30
Gl y Leu Met Val Gl y Gl y
35

<210> 2
<211> 39
<212> PRT
<213> Homo sapi ens

<400> 2
   Asp Ala Glu Phe Arg His Asp Ser Gly Tyr Glu Val His His Gln Lys
                   5                   10                  15
   Leu Val Phe Phe Ala Glu Asp Val Gly Ser Asn Lys Gly Ala Ile Ile
               20                  25                  30
   Gly Leu Met Val Gly Gly Val
           35

<210> 3
<211> 40
<212> PRT
<213> Homo sapiens

<400> 3
Asp Ala Glu Phe Arg His Asp Ser Gly Tyr Glu Val His His Gln Lys
                5                   10                  15
Leu Val Phe Phe Ala Glu Asp Val Gly Ser Asn Lys Gly Ala Ile Ile
            20                  25                  30
Gly Leu Met Val Gly Gly Val Val
        35                  40

<210> 4
<211> 41
<212> PRT
<213> Homo sapiens

<400> 4
Asp Ala Glu Phe Arg His Asp Ser Gly Tyr Glu Val His His Gln Lys
                5                   10                  15
Leu Val Phe Phe Ala Glu Asp Val Gly Ser Asn Lys Gly Ala Ile Ile
            20                  25                  30
Gly Leu Met Val Gly Gly Val Val Ile
        35                  40

<210> 5
<211> 42
<212> PRT
<213> Homo sapiens

<400> 5

<210> 6
<211> 43
<212> PRT
<213> Homo sapiens

<400> 6
Asp Ala Glu Phe Arg His Asp Ser Gly Tyr Glu Val His His Gln Lys
                5                   10                  15
Leu Val Phe Phe Ala Glu Asp Val Gly Ser Asn Lys Gly Ala Ile Ile
            20                  25                  30
Gly Leu Met Val Gly Gly Val Val Ile Ala Thr
        35                  40

<210> 7
<211> 28
<212> PRT
<213> Homo sapiens

<400> 7
Asp Ala Glu Phe Arg His Asp Ser Gly Tyr Glu Val His His Gln Lys
                5                   10                  15
Leu Val Phe Phe Ala Glu Asp Val Gly Ser Asn Lys
            20                  25

<210> 8
<211> 11
<212> PRT
<213> Homo sapiens

<400> 8

Gly Ser Asn Lys Gly Ala Ile Ile Gly Leu Met
                5                   10

<210> 9
<211> 9
<212> PRT
<213> Homo sapiens

<400> 9
Met Val Gly Gly Val Val Ile Ala Thr
                5